# EUROPEAN PATENT APPLICATION

(11) **EP 4 591 800 A1**
(43) Date of publication of application: **30.07.2025**
(21) Application number: 25153438.4
(22) Date of filing: 22.01.2025
(51) Int. Cl.: A61B 6/40, A61B 6/00

(54) **X-RAY DIAGNOSTIC APPARATUS AND METHOD OF CONTROLLING X-RAY DIAGNOSTIC APPARATUS**

(30) Priority: 23.01.2024 JP 2024008091
(71) Applicant: Canon Medical Systems Corporation, Tochigi (JP)
(72) Inventor: OKUMURA, Yusuke, Otawara-shi, 324-0036 (JP); IWAI, Haruki, Otawara-shi, 324-0036 (JP); MAEHAMA, Tomio, Otawara-shi, 324-0036 (JP); OCHIAI, Rie, Otawara-shi, 324-0036 (JP); IKEZAK, Rie, Otawara-shi, 324-0036 (JP); SOYA, Masaharu, Otawara-shi, 324-0036 (JP)
(74) Representative: Marks & Clerk LLP

(57) **Abstract**

In one embodiment, an X-ray diagnostic apparatus includes a top plate, a video system, a compensation filter, and processing circuitry. On the top plate, an object is to be placed. The video system includes an X-ray tube and an X-ray detector. The compensation filter is configured to attenuate X-rays irradiated from the X-ray tube toward the object and to be movable from a position with respect to the X-ray tube. The processing circuitry is configured to: set an initial position of the compensation filter with respect to the X-ray tube to cover a predetermined part of the object; and move a position of the compensation filter with respect to the X-ray tube from the initial position to track the predetermined part in such a manner that the compensation filter keeps covering the predetermined part of the object when at least one of the top plate and the video system is moved.

## Description

### FIELD

Embodiments disclosed in the present description and drawings relate to an X-ray diagnostic apparatus and a method of controlling the X-ray diagnostic apparatus.

### BACKGROUND

There are various kinds of X-ray diagnostic apparatus such as a general X-ray radiography apparatus and an X-ray TV apparatus. Among them, the general X-ray radiography apparatus has a relatively simple configuration and performs X-ray radiography of a chest or the like, for example. In addition, the X-ray TV apparatus is configured to be able to not only acquire X-ray radiographic images as still images but also acquire X-ray fluoroscopic images as moving images so as to execute imaging treatment using a medical device such as a catheter, that is, interventional radiology (IVR).

The X-ray diagnostic apparatus are often provided with a device that controls an X-ray irradiation region and an X-ray dosage, such as an X-ray diaphragm device and a compensation filter device, near an X-ray tube. The X-ray diaphragm device forms an aperture to transmit X-rays by a diaphragm formed of a member such as lead and irradiates only a desired region of an object with the X-rays through the aperture. On the other hand, the compensation filter device suppresses halation and suppresses exposure of the object by a compensation filter that reduces the X-rays.

The X-ray diagnostic apparatus, the X-ray TV apparatus for example, is configured such that a top plate on which an object is placed and a video system including an X-ray tube and an X-ray detector can be moved during X-ray fluoroscopy and during X-ray radiography. When a user such as a doctor or an engineer moves the top plate and the video system, fluoroscopy or radiography of a desired region of an object can be performed.

However, when the top plate or the video system is moved during the X-ray fluoroscopy and during the X-ray radiography, an aperture position of an X-ray diaphragm and a position of a compensation filter that are set beforehand are also moved. Therefore, whenever the top plate or the video system is moved, a user needs to readjust the aperture position of the X-ray diaphragm and the position of the compensation filter, and the readjustment forces operation burdens on the user.

### SUMMARY OF THE INVENTION

One problem to be solved by the embodiments disclosed in the present description and drawings is to suppress halation of X-ray images and exposure of an object while reducing operation burdens caused by adjustment of an X-ray diaphragm and a compensation filter even in a case where a top plate and a video system are moved during X-ray fluoroscopy or during X-ray radiography. However, the problem to be solved by the embodiments disclosed in the present description and drawings is not limited to the above. A problem corresponding to each effect made by each configuration shown in each embodiment to be described later may be valued as another problem to be solved.

An X-ray diagnostic apparatus according to one embodiment includes a top plate, a video system, a compensation filter, and processing circuitry. On the top plate, an object is to be placed. The video system includes an X-ray tube and an X-ray detector. The compensation filter is configured to attenuate X-rays irradiated from the X-ray tube toward the object and to be movable from a position with respect to the X-ray tube. The processing circuitry is configured to: set an initial position of the compensation filter with respect to the X-ray tube to cover a predetermined part of the object; and when at least one of the top plate and the video system is moved, move a position of the compensation filter with respect to the X-ray tube from the initial position to track the predetermined part in such a manner that the compensation filter keeps covering the predetermined part of the object.

An X-ray diagnostic apparatus according to one embodiment includes a top plate, a video system, an X-ray diaphragm, and processing circuitry. On the top plate, an object is to be placed. The video system includes an X-ray tube and an X-ray detector. The X-ray diaphragm is configured to, by forming a variable aperture, limit an irradiation range of X-rays irradiated from the X-ray tube toward the object to a range defined by the aperture and to be movable from a position with respect to the X-ray tube. The processing circuitry is configured to: set an initial position of the X-ray diaphragm with respect to the X-ray tube to irradiate a predetermined part of an object with X-rays through the aperture; and when at least one of the top plate and the video system is moved, move a position of the X-ray diaphragm with respect to the X-ray tube from the initial position to track the predetermined part in such a manner that the X-ray diaphragm keeps irradiating the predetermined part of the object with the X-rays.

Further, the processing circuitry may determine start of tracking processing based on at least one of an operation state of X-ray fluoroscopy to the object and a fluoroscopic image of the object.

Further, the processing circuitry may automatically set the initial position of the compensation filter based on a line segment set to an X-ray fluoroscopic image or an X-ray radiographic image of the object.

Further, the processing circuitry may determine a region to be covered with the compensation filter based on a luminance value of a region of the X-ray fluoroscopic image or the X-ray radiographic image divided by the line segment, and automatically set the initial position of the compensation filter to cover the region.

Further, the processing circuitry may determine an anatomical part where exposure should be reduced or a suppression part where halation should be suppressed using at least one of a result of image recognition of an X-ray fluoroscopic image or an X-ray radiographic image of the object and an imaging target part set beforehand, and set the initial position of the compensation filter to cover the determined anatomical part or suppression part with the compensation filter.

Further, the processing circuitry may detect a position of a lung base or a position of a diaphragm based on the X-ray fluoroscopic image or the X-ray radiographic image, determine a position of a lung based on the position of the lung base or the diaphragm, and set the initial position of the compensation filter to cover the determined position of the lung.

Further, the processing circuitry may set the initial position of the compensation filter based on an imaging condition set when imaging is started.

Further, the processing circuitry may detect that at least one of the top plate and the video system has been moved during a period of X-ray fluoroscopy, and start the tracking processing according to detection of the movement.

Further, the processing circuitry may start the tracking processing when the compensation filter is not manually moved for a predetermined period after X-ray fluoroscopy is started.

Further, the processing circuitry may start the tracking processing when the X-ray diaphragm is not manually moved for a predetermined period after the X-ray fluoroscopy is started.

Further, the processing circuitry may start the tracking processing when X-ray fluoroscopy is continued for a predetermined period in a state where the top plate and the video system are not moved.

Further, the processing circuitry may start the tracking processing when a pulse rate is switched in a case where the X-ray fluoroscopy is operated in a pulse fluoroscopy mode.

Further, the processing circuitry may start the tracking processing when a dosage of X-ray fluoroscopy is switched.

Further, the processing circuitry may start the tracking processing at the time of starting second X-ray fluoroscopy following first X-ray fluoroscopy when a predetermined medical device is detected in a Last Image Hold image of the first X-ray fluoroscopy.

Further, the processing circuitry may start the tracking processing at the time of starting second X-ray fluoroscopy following first X-ray fluoroscopy when a predetermined organ or tissue is detected in a Last Image Hold image of the first X-ray fluoroscopy.

Further, the processing circuitry may restart the tracking processing with a position of the compensation filter after adjustment as a new initial position in a case where the position of the compensation filter is manually adjusted during the tracking processing.

Further, the processing circuitry may stop the tracking processing in a state where the compensation filter is kept at the position when tracking stop instruction is given during the tracking processing.

Further, the processing circuitry may stop the tracking processing after withdrawing the compensation filter to a position circumscribing a visual field when tracking stop instruction is given during the tracking processing.

Further, the processing circuitry may automatically stop the tracking processing immediately or after a lapse of predetermined time in a case where the entire compensation filter goes out of a visual field during the tracking processing.

Further, the processing circuitry may perform the tracking processing by an arithmetic operation reflecting a table-to-object distance which is a distance between the predetermined part of the object and the top plate.

Further, the X-ray diagnostic apparatus may further include a display. The processing circuitry may cause the display to display an X-ray fluoroscopic image or an X-ray radiographic image of the object and a schematic diagram showing the compensation filter.

Further, the processing circuitry may display the compensation filter in different forms in the schematic diagram between a time when tracking processing is being performed and a time when the tracking processing is not being performed.

Further, the processing circuitry may display the compensation filter in the schematic diagram in a form that can be contrasted with a position of a visual field.

Further, the compensation filter may include a plurality of compensation filters. The processing circuitry may display, in a case where the compensation filter includes a plurality of compensation filters, the compensation filters in the schematic diagram which shows a relative positional relation among the plurality of compensation filters.

Further, the processing circuitry may display at least one of a moving direction of the compensation filter and a rotation radius when the compensation filter is rotationally moved in the schematic diagram.

A method of controlling an X-ray diagnostic apparatus according to one embodiment is the method of controlling the X-ray diagnostic apparatus provided with a top plate on which an object is to be placed, a video system including an X-ray tube and an X-ray detector, and a compensation filter configured to attenuate X-rays irradiated from the X-ray tube toward the object and to be movable from a position with respect to the X-ray tube. The method includes steps of: setting an initial position of the compensation filter with respect to the X-ray tube to cover a predetermined part of the object; and moving the position of the compensation filter with respect to the X-ray tube from the initial position to track the predetermined part in such a manner that the compensation filter keeps covering the predetermined part of the object when at least one of the top plate and the video system is moved.

A method of controlling an X-ray diagnostic apparatus according to one embodiment is the method of controlling the X-ray diagnostic apparatus provided with a top plate on which an object is to be placed, a video system including an X-ray tube and an X-ray detector, and an X-ray diaphragm configured to, by forming a variable aperture, limit an irradiation range of X-rays irradiated from the X-ray tube toward the object to a range defined by the aperture and to be movable from a position with respect to the X-ray tube. The method includes steps of: setting an initial position of the X-ray diaphragm with respect to the X-ray tube such that a predetermined part of an object is irradiated with X-rays through the aperture; and moving a position of the X-ray diaphragm with respect to the X-ray tube from the initial position to track the predetermined part in such a manner that the X-ray diaphragm keeps irradiating the predetermined part of the object with the X-rays when at least one of the top plate and the video system is moved.

### BRIEF DESCRIPTION OF THE DRAWINGS

Fig. 1 is a schematic diagram showing a configuration example of an X-ray diagnostic apparatus according to a first embodiment;
Fig. 2A is an explanatory diagram of an X-ray diaphragm in the first embodiment;
Fig. 2B is an explanatory diagram of a compensation filter in the first embodiment;
Fig. 3 is an explanatory diagram for an example of switches that operate the X-ray diaphragm and the compensation filter in an input interface in the first embodiment;
Fig. 4 is a flowchart showing an operation example of the X-ray diagnostic apparatus according to the first embodiment;
Fig. 5 is an explanatory diagram regarding semiautomatic setting of an initial position of the compensation filter in the first embodiment;
Fig. 6 is an explanatory diagram regarding automatic setting of the initial position of the compensation filter in the first embodiment;
Fig. 7 is an explanatory diagram regarding tracking start processing of the compensation filter in the first embodiment;
Fig. 8 is an explanatory diagram regarding tracking processing of the compensation filter when a video system is moved in a Y axis direction in the first embodiment;
Fig. 9 is an explanatory diagram regarding the tracking processing of the compensation filter when a top plate is moved in an X axis direction in the first embodiment;
Fig. 10 is an explanatory diagram regarding a TOD which is a distance between a predetermined part of an object and a top plate in the first embodiment;
Fig. 11 is an explanatory diagram regarding tracking stop processing of the compensation filter in the first embodiment;
Fig. 12 is an explanatory diagram for a first display example of a display and a mini map screen in a second embodiment;
Fig. 13 is an explanatory diagram for a second display example of the display and the mini map screen in the second embodiment;
Fig. 14 is a flowchart showing an operation example of the X-ray diagnostic apparatus according to a third embodiment; and
Fig. 15 is an explanatory diagram regarding tracking start processing of an X-ray diaphragm of the X-ray diagnostic apparatus according to the third embodiment.

### DETAILED DESCRIPTION

Hereinafter, embodiments of an X-ray diagnostic apparatus and a method of controlling the X-ray diagnostic apparatus will be described in details with reference to the drawings.

### (First Embodiment)

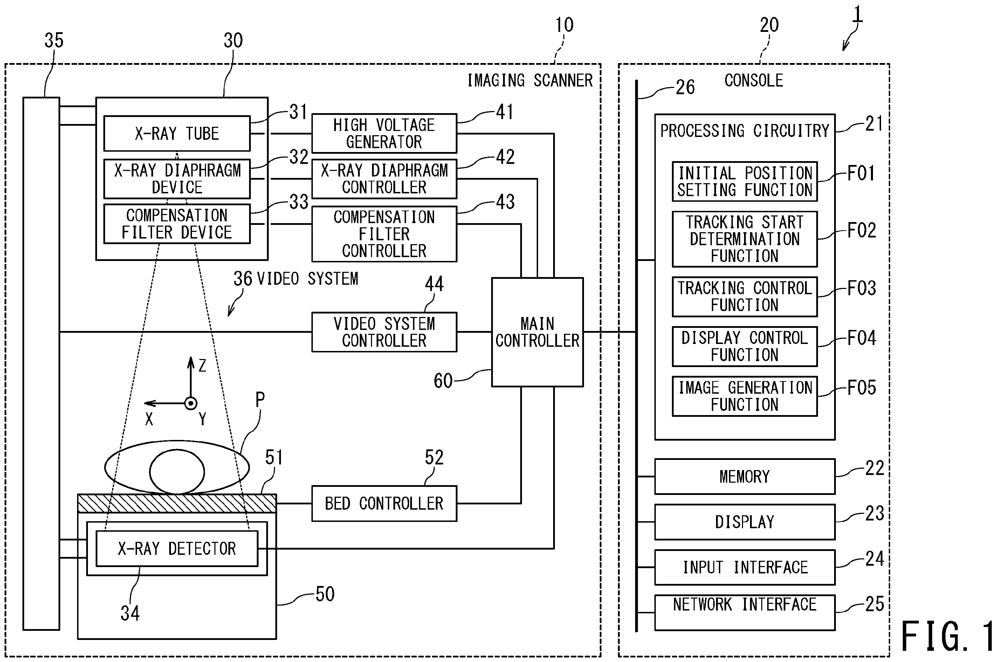

Fig. 1 is a schematic diagram showing a configuration example of an X-ray diagnostic apparatus 1 according to the first embodiment. Fig. 1 is an example of the X-ray diagnostic apparatus 1 that performs X-ray radiography of an object P in a lying position. Note that the X-ray diagnostic apparatus 1 includes a general imaging device, an X-ray round visit device, an X-ray angiographic device, an X-ray TV device, and the like. The X-ray diagnostic apparatus 1 may be, for example, an X-ray fluoroscopic diagnostic device used in a gastrointestinal contrast examination or an X-ray fluoroscopic diagnostic device for circulatory organs used in an angiography examination. Hereinafter, as shown in Fig. 1, a center axis of an X-ray irradiation direction is defined as a Z axis, an axis that is perpendicular to the Z axis and is in a longitudinal direction of a top plate 51 is defined as a Y axis, and an axis that is perpendicular to the Z axis and the Y axis and is in a short direction of the top plate 51 is defined as an X axis.

As shown in Fig. 1, the X-ray diagnostic apparatus 1 includes an imaging scanner 10, and a console 20 as an example of an image processor. The imaging scanner 10 includes an X-ray irradiation device 30, an X-ray detector 34, a support frame 35, and a bed 50.

The X-ray irradiation device 30 includes an X-ray tube 31, an X-ray diaphragm device 32, and a compensation filter device 33. The X-ray tube 31 is a vacuum tube that emits thermions from a cathode (filament) to an anode (target) by application of a high voltage from a high voltage generator 41. The object P is irradiated with X-rays generated by the X-ray tube 31.

The X-ray diaphragm device 32 is provided between the X-ray tube 31 and the object P, and forms an irradiation range of X-rays on a detection surface of the X-ray detector 34. The X-ray diaphragm device 32 includes a plurality of independently movable diaphragm blades. The plurality of diaphragm blades provided in the X-ray diaphragm device 32 are also referred to simply as an X-ray diaphragm. By forming a variable aperture, the X-ray diaphragm limits the irradiation range of the X-rays irradiated from the X-ray tube 31 toward the object P to a range defined by the aperture. Further, the X-ray diaphragm device 32 is configured to be movable from a position with respect to the X-ray tube 31. An X-ray diaphragm controller 42 can move the aperture of the X-ray diaphragm in order to adjust the irradiation range of the X-rays. The plurality of diaphragm blades are, for example, formed of lead that shields X-rays, and are movable in an X axis direction and a Y axis direction that are orthogonal to the X-ray irradiation direction.

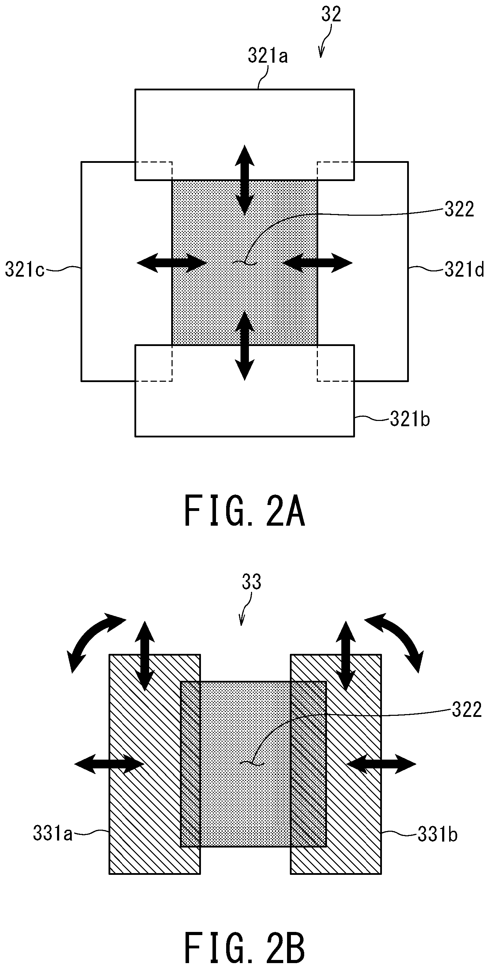

Fig. 2A is an example of the X-ray diaphragm. In Fig. 2A, the X-ray diaphragm device 32 includes a pair of diaphragm blades 321c and 321d that narrows the X-rays spreading in the X axis direction, and a pair of diaphragm blades 321a and 321b that narrows the X-rays spreading in the Y axis direction. The X-ray diaphragm controller 42 may asymmetrically control the diaphragm blades of the X-ray diaphragm device 32 in each direction, or may symmetrically control the pairs of the diaphragm blades. For example, the X-ray diaphragm controller 42 may control all the diaphragm blades such that each blade moves independently, or may control the two pairs of the diaphragm blades such that each pair move laterally symmetrically or vertically symmetrically, respectively. In addition, the X-ray diaphragm may have a multilayer structure provided with the plurality of diaphragm blades in the Z axis direction.

The compensation filter device 33 is provided between the X-ray diaphragm device 32 and the object P, and includes one or more independently movable compensation filters. The compensation filter is configured to attenuate the X-rays irradiated from the X-ray tube 31 toward the object P and to be movable from a position with respect to the X-ray tube 31. A compensation filter controller 43 can move the position of the compensation filter with respect to the X-ray tube 31. The compensation filter is formed of a metal plate or the like that has a rectangular shape, a semicircular shape or an elliptic shape, for example, and is movable in a direction orthogonal to the X-ray irradiation direction.

Fig. 2B is an example of the compensation filter device 33. In Fig. 2B, the compensation filter device 33 includes two compensation filters 331a and 331b. The compensation filters 331a and 331b are disposed such that longitudinal directions of the rectangular shapes of the compensation filters 331a and 331b are parallel to each other as an initial state, for example. By moving both ends of the compensation filters 331a and 331b by an equal distance, the compensation filter device 33 can parallelly move them in the X axis direction or the Y axis direction orthogonal to the X-ray irradiation direction. In addition, by moving both ends of the compensation filters 331a and 331b by distances different from each other, the compensation filter device 33 can rotationally move them in a 0 direction or a ϕ direction on a plane orthogonal to the X-ray irradiation direction. Further, while the two compensation filters are provided in Fig. 2B, a number of the compensation filters may be one or may be three or more.

The X-ray detector 34 is provided with, for example, a plane detector (FPD) including a plurality of two-dimensionally arrayed X-ray detection elements, and an analog/digital converter (A/D converter) that converts electric signals to digital data. The X-ray detector 34 detects the X-rays that are generated from the X-ray tube 31, irradiated to the object P, and transmitted through the object P. The X-ray detector 34 supplies, to the console 20, X-ray image data such as fluoroscopic imaging data generated by X-ray fluoroscopy of imaging time-sequentially continuous X-ray images (frame images) in real time or supplies simple imaging data generated by simple X-ray radiography of imaging one X-ray image, based on the detected X-rays.

The support frame 35 movably supports the X-ray irradiation device 30 and the X-ray detector 34 that are disposed facing each other. Here, a configuration including at least the X-ray tube 31 and the X-ray detector 34 is referred to as a video system 36. The video system 36 may also include the X-ray irradiation device 30, the X-ray detector 34, and the support frame 35. A video system controller 44 can move the video system 36 in a longitudinal direction (Y axis direction) of the bed 50, for example. Further, the video system controller 44 can move the X-ray irradiation device 30 in a vertical direction.

The bed 50 is supported by a floor surface, and includes the top plate 51 on which the object P is to be placed. The top plate 51 is also referred to as a decubitus table, and the object P is placed thereon. The object P is disposed between the X-ray irradiation device 30 and the X-ray detector 34 in the X-ray radiography. A bed controller 52 can move the top plate 51 on which the object P is placed in the short direction (X axis direction), for example, with respect to a main body of the bed 50 housing the X-ray detector 34. Further, the bed controller 52 is capable of sliding (in the X and Y axis directions), vertically moving (in the Z axis direction), and rolling the bed 50.

A main controller 60 includes at least a central processing unit (CPU) and a memory that are not shown, and is controlled by processing circuitry 21 of the console 20 to generally control respective components such as the high voltage generator 41, the X-ray diaphragm controller 42, the compensation filter controller 43, the video system controller 44, and the bed controller 52 of the imaging scanner 10.

The console 20 as an example of the image processor has the processing circuitry 21, a memory 22, a display 23, an input interface 24, and a network interface 25. The console 20 does not need to be independently provided, and a processor and a memory in the main controller 60 of the imaging scanner 10 may respectively be in charge of functions of the processing circuitry 21 and the memory 22.

The network interface 25 is mounted with various information communication protocols according to a network form. The network interface 25 performs communication control according to the various kinds of protocols, and can be connected to a network via a cable or radio. The network interface 25 can exchange various kinds of data between the network and the memory 22, for example.

The input interface 24 includes an input device that can be operated by a user, and an input circuit that receives signals from the input device. The input device is realized by, for example, an operator console, a joystick, a trackball mouse, a keyboard, a touch panel with which an input operation is performed by touching an operation surface, a touch screen for which a display screen and a touch pad are integrated, a non-contact input circuit using an optical sensor, and a voice input circuit.

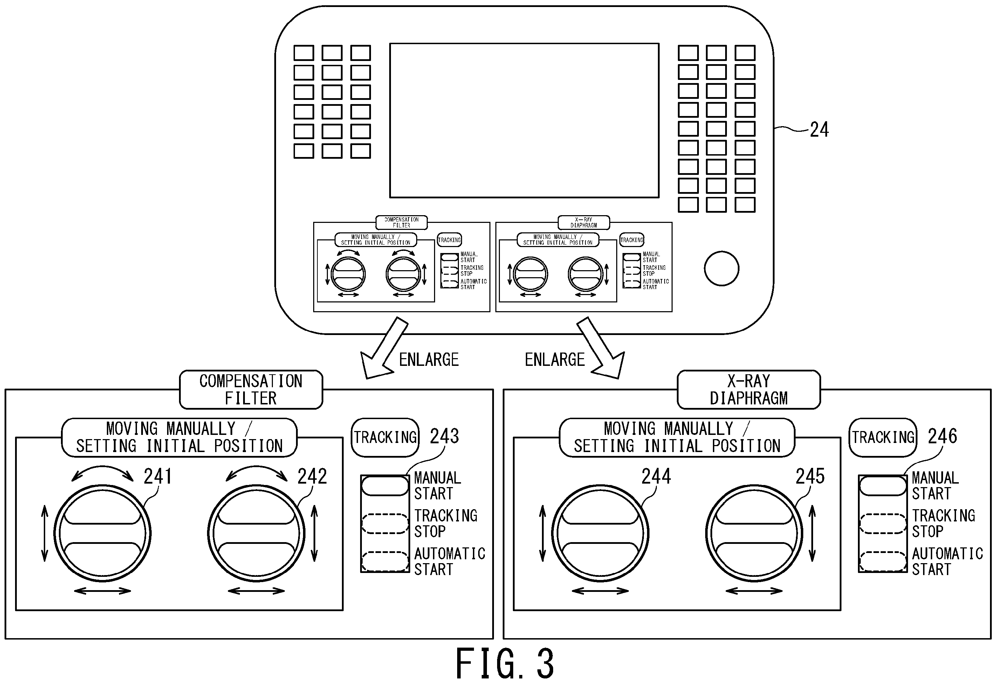

Here, with reference to Fig. 3, an example of switches that are attached to the input interface 24 to operate the X-ray diaphragm and the compensation filter will be described. When the top plate 51 or the video system 36 is moved during the X-ray fluoroscopy or during the X-ray radiography, an aperture position of the X-ray diaphragm and a position of the compensation filter that are set beforehand are also moved. Therefore, according to the top plate 51 or the video system 36 is moved, the user needs to readjust the position of the X-ray diaphragm and the position of the compensation filter.

The input interface 24 is provided with switches for controlling movement of the compensation filter 331 and the X-ray diaphragm.

Switches 241 and 242 for setting the position of the compensation filters are knobbed switches respectively, and when the user performs back and forth, left and right, and rotating operations of a knob, the compensation filter can be manually moved. When the user performs the back and forth, left and right and rotating operations of the knobs of the switches 241 and 242 for setting the position of the compensation filters, according to the operation amount, the compensation filters 331a and 331b in Fig. 2B are moved back and forth and left and right and are rotated. By performing the back and forth, left and right, and rotating operations of the knobs of the switches 241 and 242, an initial position of a tracking operation of the compensation filters can be manually set.

While Fig. 3 shows a case where the two compensation filters are provided and the switches 241 and 242 for setting the position of the two compensation filters are provided so as to perform a manual operation for each compensation filter, the number of the switches for setting the position of the compensation filter is not limited. The number of the switches for setting the position of the compensation filter may be one or may be three or more according to the number of the compensation filters.

In addition, a switch 243 for tracking of the compensation filter is a slide type switch capable of being set to manual start, tracking stop and automatic start, and when the user slides and sets the switch 243 to one of the modes of the manual start, the tracking stop, and the automatic start, an operation mode of tracking of the compensation filter is set according to the setting operation.

Switches 244 and 245 for setting the position of the X-ray diaphragm are also knobbed switches respectively, for example, and when the user performs an operation of moving the knob back and forth and left and right, the X-ray diaphragm can be manually moved. When the user performs the operation of moving the knob of the switch 244 for setting the position of the X-ray diaphragm back and forth and left and right, according to the operation amount, the diaphragm blades 321a and 321c in Fig. 2A are opened or closed to front and back and left and right. When the user performs an operation of moving the knob of the switch 245 for setting the position of the X-ray diaphragm back and forth and left and right, according to the operation amount, the diaphragm blades 321b and 321d in Fig. 2A are opened or closed to the front and back and left and right. Further, when the user performs the operation of moving the knobs of the switches 244 and 245 back and forth and left and right, an initial position of a tracking operation of the X-ray diaphragm can be manually set.

While Fig. 3 shows two switches for setting the position of the X-ray diaphragm, the number of the switches for the X-ray diaphragm is not limited. The number of the switches for setting the position of the X-ray diaphragm may be one or may be three or more. When one switch for setting the position of the X-ray diaphragm is provided, the X-ray diaphragm may be moved back and forth and left and right in a state where the aperture of the X-ray diaphragm is maintained.

Further, a switch 246 for tracking of the X-ray diaphragm is a slide type switch capable of being set to manual start, tracking stop and automatic start, and when the user slides and sets the switch 246 to one of the modes of the manual start, the tracking stop and the automatic start, an operation mode of tracking of the X-ray diaphragm is set according to the operation.

Note that setting of the modes of the manual start, the tracking stop and the automatic start is not limited to the switch 243 for the tracking of the compensation filter and the switch 246 for the tracking of the X-ray diaphragm described above. For example, setting may be performed with a hard button or a soft button disposed on the console 20. In addition, setting may be performed by pressing an existing button twice or for a long time without disposing a new exclusive button. Setting may be performed by pushing in the switches 241 and 242 for setting the position of the compensation filter and pushing in the switches 244 and 245 for setting the position of the X-ray diaphragm. Further, the manual start, the tracking stop and the automatic start may be set with voice recognition or visual line movement of the user as a trigger.

The display 23 is formed of, for example, a general display output device such as a liquid crystal display and an organic light emitting diode (OLED) display. The display 23 displays an X-ray fluoroscopic image, an X-ray radiographic image, and a mini map or the like generated according to control of the processing circuitry 21. The mini map is an example of a schematic diagram. The schematic diagram may be a diagram that schematically displays the compensation filter, and is not limited to the mini map.

For the display 23, an entire surface of the display may be a display window, a part of the display may be a display window, and it may be switchable between both. On the display 23, the X-ray fluoroscopic image or the X-ray radiographic image and the mini map may be displayed side by side in the display window. The display 23 may further display a screen regarding patient information and various kinds of input.

The memory 22 is formed of a storage medium readable by a processor, such as a semiconductor memory element like a random access memory (RAM) and a flash memory, a hard disk, and an optical disk, for example. The memory 22 may be formed of a portable medium such as a universal serial bus (USB) memory and a digital video disk (DVD), for example. The memory 22 stores various kinds of processing programs used in the processing circuitry 21, and data or the like needed to execute the programs. In addition, the memory 22 stores various kinds of data such as an imaging condition regarding the X-ray radiography or the X-ray fluoroscopy, and image data of the X-ray fluoroscopic image or the X-ray radiographic image or the like.

The processing circuitry 21 has a processor, and realizes respective functions to be described later by software processing by executing the programs stored in the memory 22. Further, the processing circuitry 21 generally controls the respective components of the imaging scanner 10 via the main controller 60.

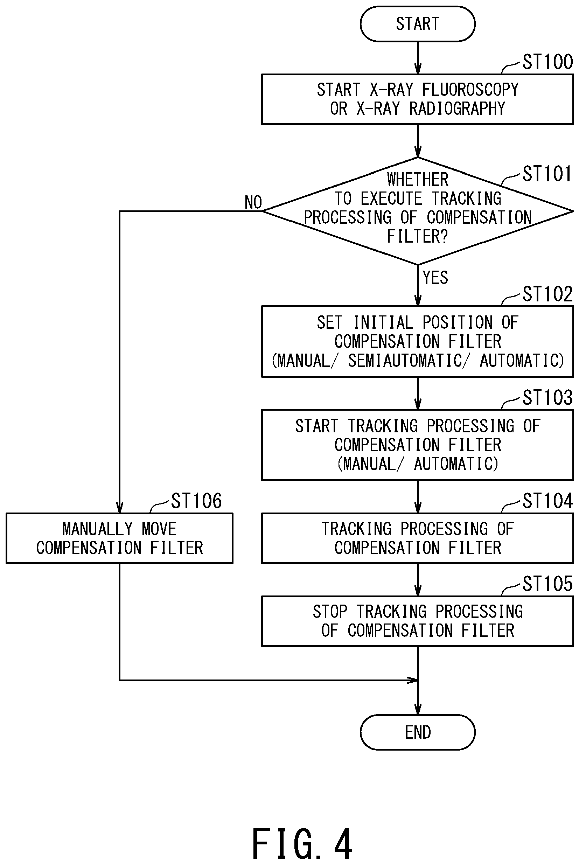

As shown in Fig. 1, the processing circuitry 21 realizes the respective functions that are an initial position setting function F01, a tracking start determination function F02, a tracking control function F03, a display control function F04, and an image generation function F05. Note that the display control function F04 is not essential, and is to be described later in a second embodiment. Operation examples of the respective functions of the processing circuitry 21 will be described with reference to a flowchart in Fig. 4 and Fig. 5 to Fig. 11.

In step ST100, the X-ray fluoroscopy or the X-ray radiography is started. For example, the X-ray fluoroscopy or the X-ray radiography is performed based on the imaging condition specified by the user via the input interface 24 and the imaging condition stored in the memory 22 beforehand according to a diagnostic part of the object P, examination contents, an examination purpose, and an examination protocol or the like. The image generation function F05 generates the X-ray fluoroscopic image or the X-ray radiographic image based on X-ray data detected in the X-ray detector 34.

In step ST101, whether or not to execute tracking processing of the compensation filter 331 is selected. For example, selection is made by the user via the input interface 24.

In the case where the tracking processing of the compensation filter is to be executed (that is, in the case of YES) in step ST101, processing advances to step ST102. In the case where the tracking processing of the compensation filter is not to be executed (that is, in the case of NO) in step ST101, the processing advances to step ST106. In step ST106, the user manually moves the compensation filter via the switches 241 and 242 provided for setting the position of the compensation filter in the input interface 24, for example.

In step ST102, the initial position setting function F01 sets an initial position of the compensation filter with respect to the X-ray tube 31 so as to cover a predetermined part of the object P. The initial position of the compensation filter may be manually set, semiautomatically set, or automatically set. In the case of manually setting the initial position for the tracking, the user manually sets the initial position of the compensation filter via the switches 241 and 242 provided for setting the position of the compensation filter in the input interface 24, for example. The user manually disposes the compensation filter at an anatomically appropriate position under the X-ray fluoroscopy, for example.

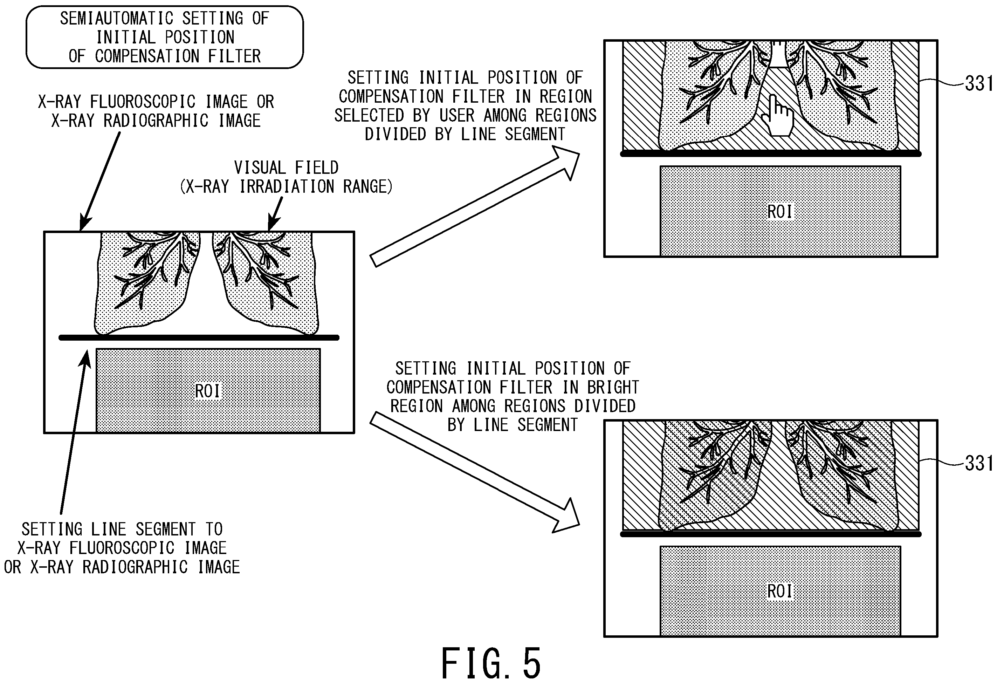

Fig. 5 is an explanatory diagram for the semiautomatic setting of the initial position of the compensation filter. Fig. 5 is an examination example having a lung included in an X-ray image, and a region of interest (ROI) is a region excluding the lung. As shown in Fig. 5, for example, the user sets a line segment to the X-ray fluoroscopic image or the X-ray radiographic image obtained by imaging the object P.
The user may set the line segment using the mouse of the input interface 24, for example. In addition, the user may change a position of the line segment by using a GUI function capable of moving and rotating the line segment set to the X-ray fluoroscopic image or the X-ray radiographic image.

The user can semiautomatically set the position of the compensation filter by utilizing the set line segment. For example, the user may select one of regions divided by the line segment, and the initial position of the compensation filter may be set in the region selected by the user. The user may select one of the regions divided by the line segment as a region to dispose the compensation filter by double-clicking the mouse or the touch panel of the input interface 24, for example.

The initial position of the compensation filter may be determined according to the imaging condition set when imaging is started. When the compensation filter is disposed beforehand at a suitable position according to the imaging condition, slight adjustment to movement of the compensation filter is sufficient. On the other hand, the compensation filter does not need to be imaged in the X-ray fluoroscopic image or the X-ray radiographic image before the compensation filter is disposed. That is, the compensation filter does not need to be set before the X-ray fluoroscopy or the X-ray radiography is started.

Further, the initial position setting function F01 may automatically set the initial position of the compensation filter based on the line segment set to the X-ray fluoroscopic image or the X-ray radiographic image obtained by imaging the object P. For example, the initial position setting function F01 may analyze and determine a region to be covered with the compensation filter based on a luminance value of a region of the X-ray fluoroscopic image or the X-ray radiographic image divided by the line segment, and may automatically set the initial position of the compensation filter so as to cover the region. In Fig. 5, the initial position setting function F01 automatically sets the initial position of the compensation filter such that the compensation filter covers a bright region. In the case where black and white of the X-ray fluoroscopic image or the X-ray radiographic image are inverted and displayed, the initial position setting function F01 may automatically set the initial position of the compensation filter such that the compensation filter covers a dark region.

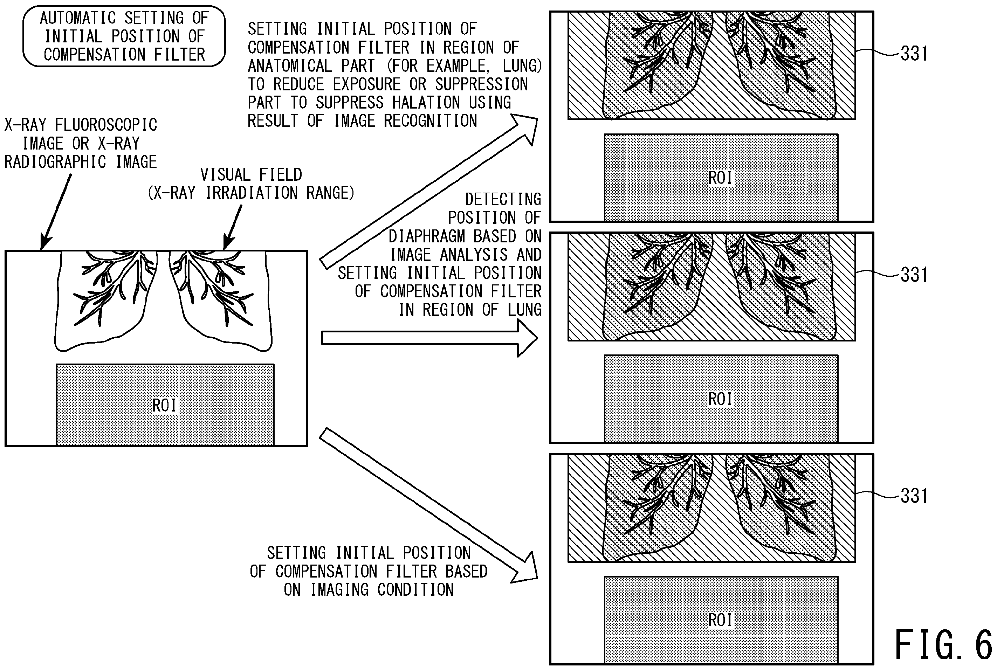

Fig. 6 is an explanatory diagram regarding automatic setting of the initial position of the compensation filter. As shown in Fig. 6, the initial position setting function F01 determines an anatomical part where exposure should be reduced or a suppression part where halation should be suppressed, by using at least one of a result of image recognition to the X-ray fluoroscopic image or the X-ray radiographic image obtained by imaging the object P and an imaging target part set beforehand based on the imaging condition, and sets the initial position of the compensation filter such that the compensation filter covers the determined anatomical part or suppression part. Note that the image recognition to the X-ray fluoroscopic image or the X-ray radiographic image obtained by imaging the object P, that is, recognition of the anatomical part or the suppression part may be performed using segmentation and artificial intelligence (AI) or the like.

The initial position of the compensation filter may be set according to the anatomical part or the suppression part. For example, the initial position setting function F01 may detect a position of a lung base or a position of a diaphragm based on the X-ray fluoroscopic image or the X-ray radiographic image, determine a position of a lung based on the position of the lung base or the diaphragm, and set the initial position of the compensation filter such that the compensation filter covers the determined position of the lung.

Further, the initial position setting function F01 may set the initial position of the compensation filter based on the imaging condition set when the imaging is started.

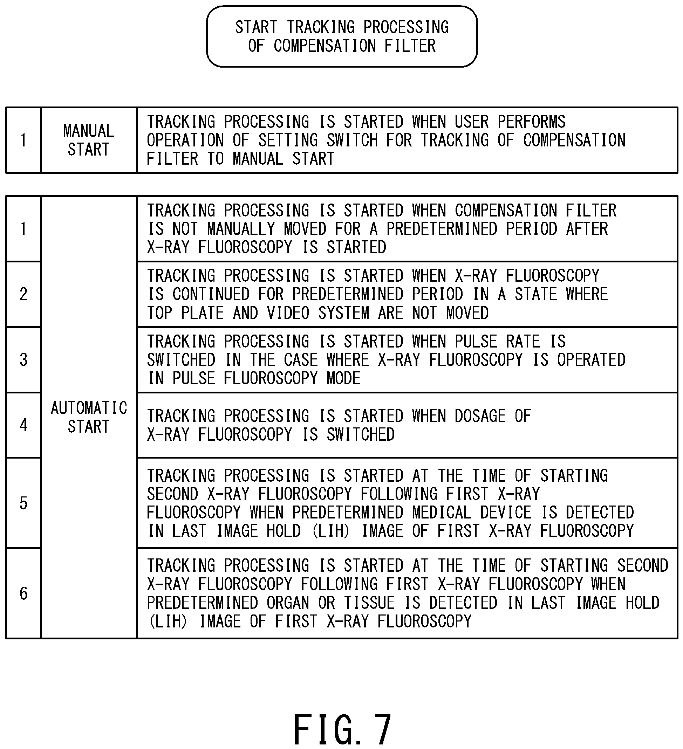

In step ST103, the tracking start determination function F02 starts the tracking processing of the compensation filter. The tracking start determination function F02 determines start of the tracking processing based on at least one of an operation state of the X-ray fluoroscopy to the object P and a fluoroscopic image obtained by imaging the object P. Tracking start processing of the compensation filter will be described with reference to Fig. 7. The tracking processing of the compensation filter may be manually started or may be automatically started.

In the case of the manual start, for example, when the user performs an operation of setting the switch 243 for the tracking of the compensation filter 331 to the manual start, the tracking processing of the compensation filter 331 is started (manual start 1 in Fig. 7).

In the case of the automatic start, when the tracking start determination function F02 detects that at least one of the top plate 51 and the video system 36 has been moved during a period of the X-ray fluoroscopy, it starts the tracking processing according to detection of the movement. For example, the tracking start determination function F02 may start the tracking processing when the compensation filter is not manually moved for a predetermined period after the X-ray fluoroscopy is started (automatic start 1 in Fig. 7). The tracking start determination function F02 may start the tracking processing when the X-ray fluoroscopy is continued for the predetermined period in a state where the top plate 51 and the video system 36 are not moved (automatic start 2 in Fig. 7).

The tracking start determination function F02 may start the tracking processing when a pulse rate is switched in the case where the X-ray fluoroscopy is operated in a pulse fluoroscopy mode (automatic start 3 in Fig. 7). For example, a time when the pulse rate is switched may be determined as a timing that the imaging starts, such as the case where positioning is performed at a low pulse rate and then switched to a high pulse rate when performing imaging, or the case where positioning is performed at a pulse rate initially set according to the imaging condition and then switched to a low pulse rate when performing imaging in order to reduce the exposure or the like. Then, the tracking start determination function F02 may start the tracking processing.

The tracking start determination function F02 may start the tracking processing when a dosage of the X-ray fluoroscopy is switched (automatic start 4 in Fig. 7). For example, a time when the dosage of the X-ray fluoroscopy is switched may be determined as the timing that the imaging starts, such as the case where the X-ray fluoroscopy is started at low dosage and then switched to high dosage when performing imaging, or the case where the X-ray fluoroscopy is started at dosage initially set for a predetermined examination and then switched to low dosage when performing imaging in order to reduce the exposure or the like. Then, the tracking start determination function F02 may start the tracking processing.

In addition, when a predetermined medical device is detected in a last image hold (LIH) image of first X-ray fluoroscopy, the tracking start determination function F02 may start the tracking processing at the time of starting second X-ray fluoroscopy following the first X-ray fluoroscopy (automatic start 5 in Fig. 7). Further, when a predetermined organ or tissue is detected in the LIH image of the first X-ray fluoroscopy, the tracking start determination function F02 may start the tracking processing at the time of starting the second X-ray fluoroscopy following the first X-ray fluoroscopy (automatic start 6 in Fig. 7).

When tracking is started in step ST103, in next step ST104, the tracking processing of the compensation filter is executed. When at least one of the top plate 51 and the video system 36 is moved, the tracking control function F03 moves the position of the compensation filter with respect to the X-ray tube 31 from the initial position to track the predetermined part of the object P such that the compensation filter keeps covering the predetermined part.

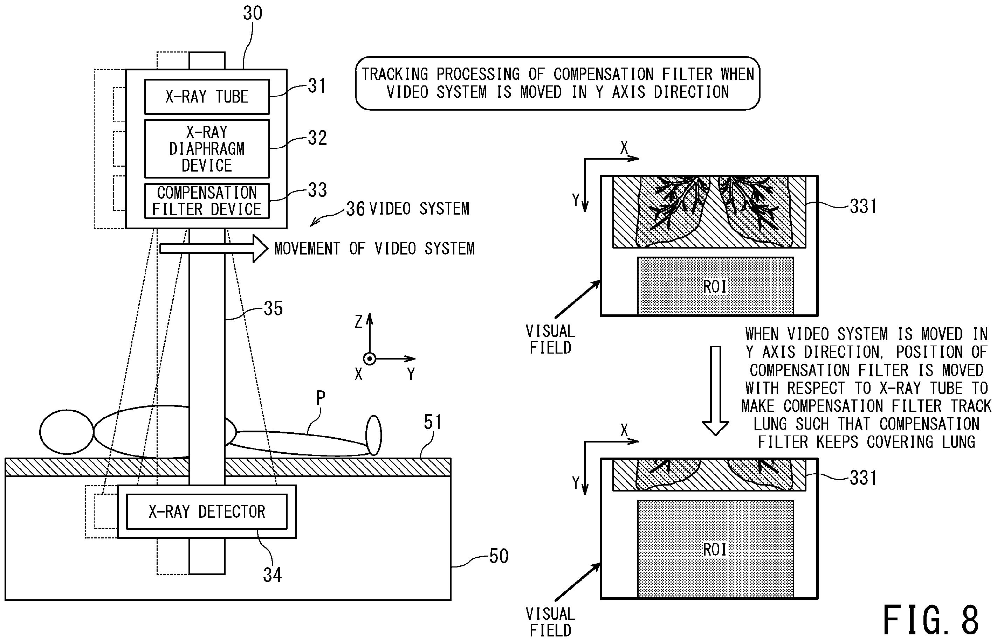

Fig. 8 is an explanatory diagram regarding the tracking processing of the compensation filter 331 when the video system 36 is moved in the Y axis direction. As shown in Fig. 8, in the case where the compensation filter 331 is initially set at the lung, when the video system 36 is moved in the Y axis direction, the tracking control function F03 moves the position of the compensation filter 331 with respect to the X-ray tube 31 to make the compensation filter 331 track the lung such that the compensation filter 331 keeps covering the lung.

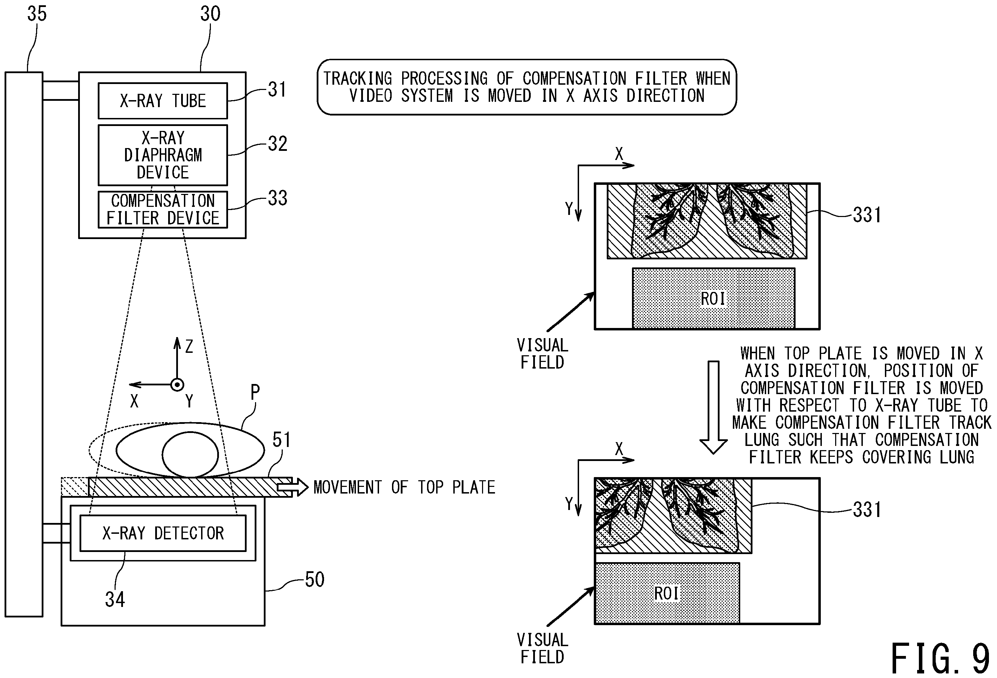

Fig. 9 is an explanatory diagram regarding the tracking processing of the compensation filter 331 when the top plate 51 is moved in the X axis direction. As shown in Fig. 9, in the case where the compensation filter 331 is initially set at the lung, when the top plate 51 is moved in the X axis direction, the tracking control function F03 moves the position of the compensation filter 331 with respect to the X-ray tube 31 to make the compensation filter 331 track the lung such that the compensation filter 331 keeps covering the lung.

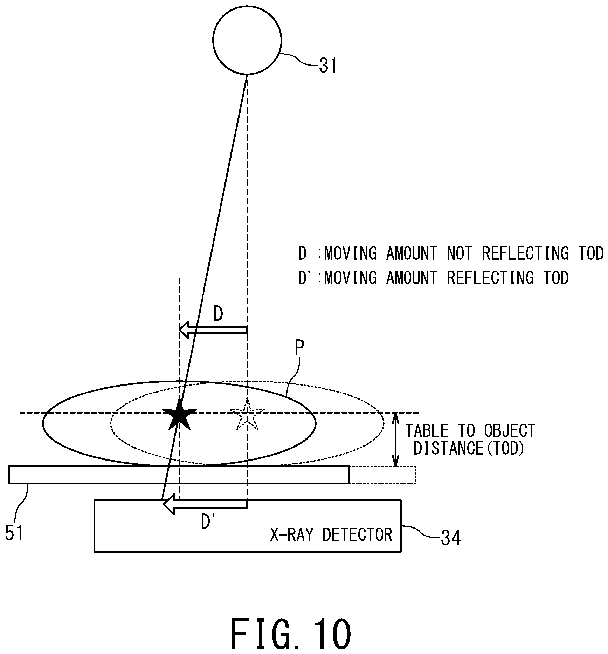

Further, the tracking control function F03 may perform the tracking processing by an arithmetic operation reflecting a table-to-object distance (TOD) which is a distance between the predetermined part of the object P and the top plate 51. Fig. 10 is an explanatory diagram regarding the TOD which is the distance between the predetermined part of the object P indicated with a star mark and the top plate 51. As shown in Fig. 10, a moving amount D' reflecting the TOD is calculated based on an angle at which the X-rays irradiated from the X-ray tube 31 and enter the X-ray detector 34. Further, the moving amount D' reflecting the TOD can be calculated by considering rotation of the video system 36 as well in addition to the movement in the X axis direction and in the Y axis direction. Compared to the case of calculating a moving amount D (the moving amount not reflecting the TOD) on the X-ray detector 34 based on merely the moving amount of the top plate 51 of the video system 36, the tracking processing with higher accuracy is made possible by calculating the moving amount D' reflecting the TOD.

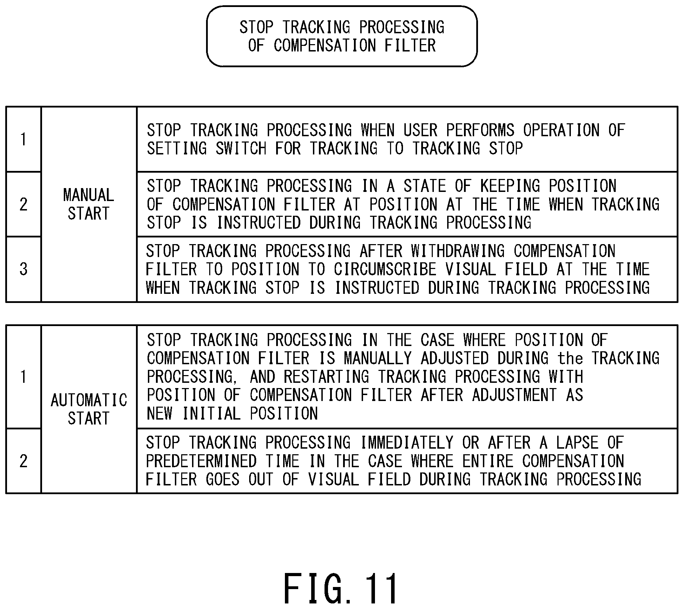

In step ST105, the tracking control function F03 stops the tracking processing of the compensation filter. Fig. 11 is an explanatory diagram regarding manual stop and automatic stop of the tracking processing of the compensation filter.

The tracking processing of the compensation filter may be manually stopped. For example, when the user performs an operation of setting the switch 243 provided for the tracking of the compensation filter 331 to the tracking stop, the tracking processing of the compensation filter 331 is stopped (manual stop 1 in Fig. 11). When the tracking stop instruction is given during the tracking processing, the tracking control function F03 may stop the tracking processing in a state where the compensation filter is kept at that position when being instructed (manual stop 2 in Fig. 11). When the tracking stop instruction is given during the tracking processing, the tracking control function F03 may stop the tracking processing after withdrawing the compensation filter to a position circumscribing a visual field (that is, the X-ray irradiation range) at the time (manual stop 3 in Fig. 11).

Further, the tracking processing of the compensation filter may be automatically stopped. For example, in the case where the position of the compensation filter is manually adjusted during the tracking processing, the tracking control function F03 stops the tracking processing. In this case, the tracking processing may be restarted with the position of the compensation filter after the adjustment as a new initial position (automatic stop 1 in Fig. 11). Also, the position of the compensation filter before the adjustment may be stored such that if a new initial position is erroneously set, such position can be restored. In addition, in the case where the entire compensation filter goes out of the visual field during the tracking processing, the tracking control function F03 may automatically stop the tracking processing immediately, or after a lapse of predetermined time (automatic stop 2 in Fig. 11).

The X-ray diagnostic apparatus 1 according to the first embodiment makes it possible to suppress the halation of the X-ray images and the exposure of the object while reducing operation burdens caused by the adjustment of the compensation filter even if the top plate and the video system are moved during the X-ray fluoroscopy or during the X-ray radiography.

### (Second Embodiment)

In the X-ray diagnostic apparatus 1 according to the second embodiment, in addition to the first embodiment, the display control function F04 displays the X-ray fluoroscopic image or the X-ray radiographic image obtained by imaging the object P and the mini map that schematically displays the compensation filter on the display 23. Other configurations and functions are not practically different from those in the X-ray diagnostic apparatus 1 according to the first embodiment so redundant descriptions will be omitted.

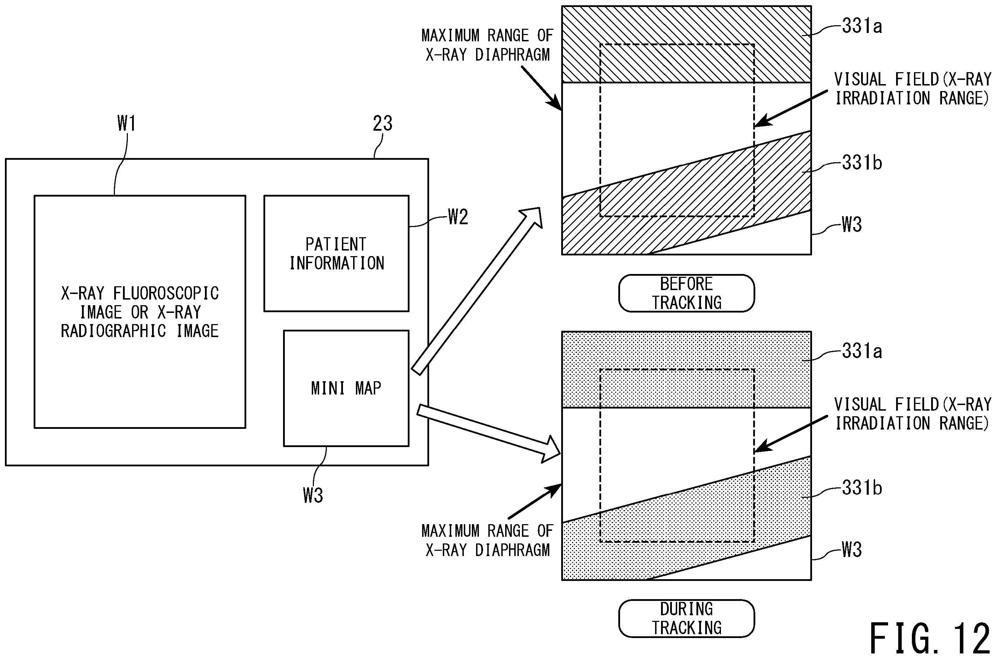

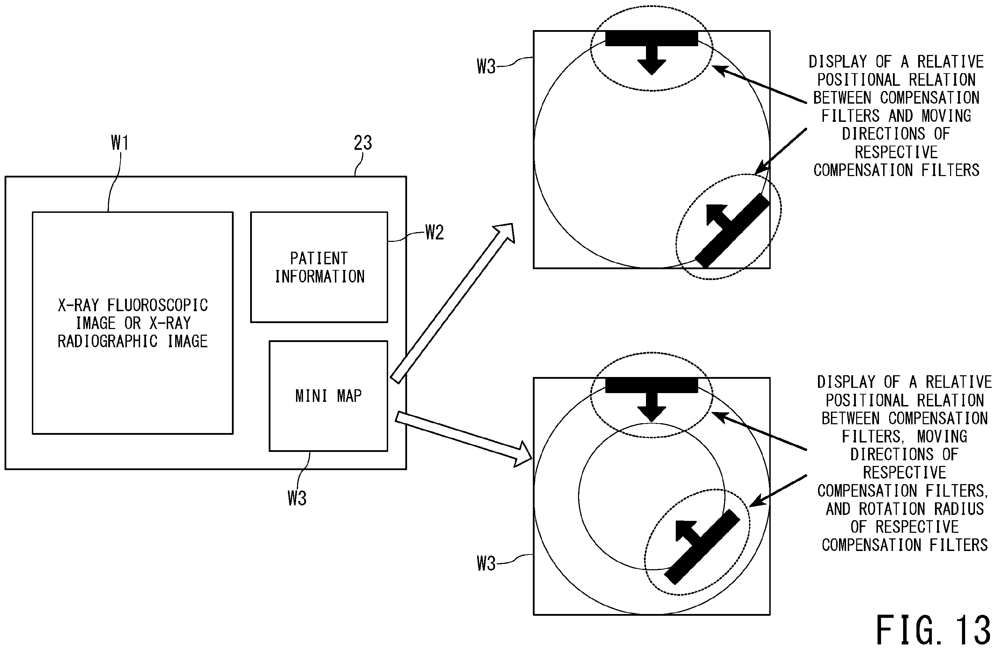

Fig. 12 and Fig. 13 are explanatory diagrams showing a display example of the display 23 and a mini map screen W3. As shown in the display example of the display 23 shown on a left side of Fig. 12 and Fig. 13, the mini map screen W3 is displayed on the display 23 side by side with a screen W1 of the X-ray fluoroscopic image or the X-ray radiographic image and a screen W2 of patient information, for example.

On the mini map screen W3 in Fig. 12, ranges where the compensation filters 331a and 331b are disposed are superimposed and displayed on a maximum range of the X-ray diaphragm. In this case, the display control function F04 may display the compensation filters 331a and 331b on the mini map in different forms between the time when the tracking processing is being performed and the time when it is not being performed. For example, as the compensation filters 331a and 331b in Fig. 12 before the tracking and during the tracking are indicated by different kinds of hatching, whether or not it is during automatic tracking may be displayed using different colors and flickering or the like, such that the user can make judgement easily.

Further, the display control function F04 may have the compensation filters 331a and 331b displayed on the mini map in a form to be contrasted with the position of the visual field. On the mini map screen W3 in Fig. 12, the position of the visual field is displayed with a broken line. By displaying the position of the visual field with the broken line, even in the case where the position of the compensation filter goes out of the visual field, it can be confirmed in the maximum range of the X-ray diaphragm.

Further, the mini map screen W3 may be utilized in an automatic tracking establishing operation. For example, when the user drags the compensation filter displayed on the mini map screen W3 to a position where the user wants to dispose it with the mouse or clicks the compensation filter that the user wants to move and then clicks the position where the user wants to dispose it, the position of the compensation filter may be moved on the mini map screen W3.

On the mini map screen W3 shown at upper right in Fig. 13, a relative positional relation between the two compensation filters 331a and 331b is displayed without expressing a rotation radius at the time of rotational movement. In addition, directions of arrows in Fig. 13 indicate moving directions in the case of manually operating the respective compensation filters 331a and 331b.

On the mini map screen W3 shown at lower right in Fig. 13, in addition to the relative positional relation between the two compensation filters 331a and 331b and the moving directions of the respective compensation filters 331a and 331b, the rotation radius when the respective compensation filters 331a and 331b are rotationally moved is expressed and displayed. In such a manner, in the case where the plurality of compensation filters is provided, the display control function F04 may display the compensation filters on the mini map such that the relative positional relation among the plurality of compensation filters can be recognized.

The mini map screen W3 may express and display the relative positional relation between the two compensation filters 331a and 331b and the rotation radius when the respective compensation filters 331a and 331b are rotationally moved. In such a manner, the display control function F04 may display at least one of the moving direction of the compensation filter and the rotation radius when the compensation filter is rotationally moved on the mini map.

The X-ray diagnostic apparatus 1 according to the second embodiment makes it possible to obtain equivalent effects to that of the X-ray diagnostic apparatus 1 according to the first embodiment. Further, since the mini map is displayed side by side with the X-ray fluoroscopic image or the X-ray radiographic image in the second embodiment, it is easier for the user to recognize the positions of the respective compensation filters with respect to the position of the visual field, the relative positional relation among the plurality of compensation filters, the moving directions of the respective compensation filters, and the rotation radius altogether without disturbing observation of the X-ray fluoroscopic image or the X-ray radiographic image.

### (Third Embodiment)

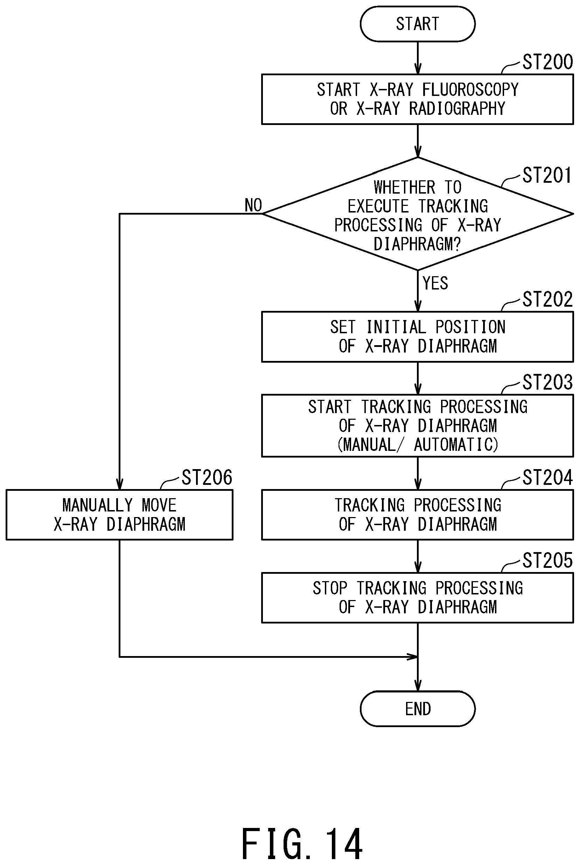

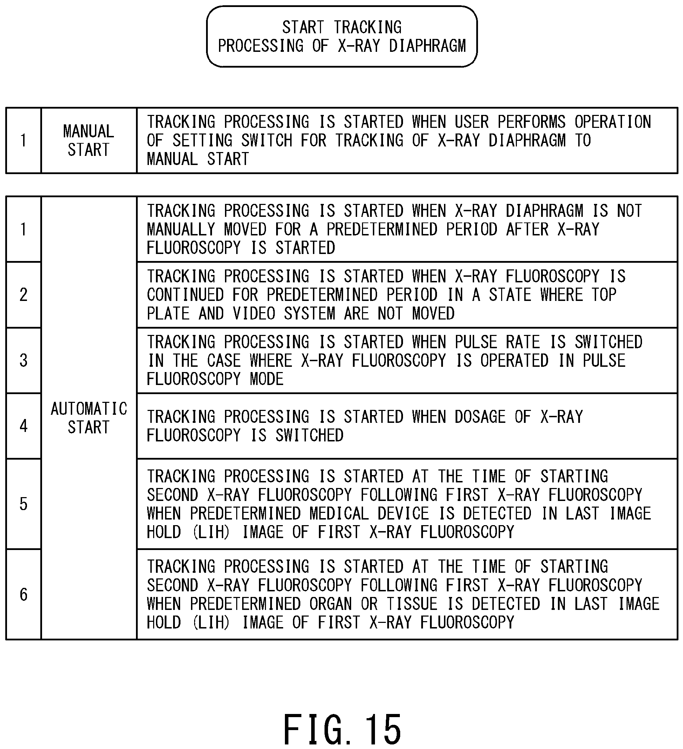

The X-ray diagnostic apparatus 1 according to the first embodiment suppresses the halation of the X-ray images and the exposure of the object by making the compensation filter perform tracking in the case where the top plate and the video system are moved during the X-ray fluoroscopy or during X-ray radiography. The X-ray diagnostic apparatus 1 according to the third embodiment differs from the first embodiment in that the X-ray diaphragm is made to perform tracking in place of the compensation filter. The other configurations and functions are not practically different from those in the X-ray diagnostic apparatus 1 according to the first embodiment shown in Fig. 1 so the redundant descriptions will be omitted. Operation examples of the respective functions of the processing circuitry 21 will be described with reference to a flowchart in Fig. 14 and Fig. 15.

In step ST200, the X-ray fluoroscopy or the X-ray radiography is started. Step ST200 in the third embodiment is not practically different from step ST100 in the first embodiment so the redundant description will be omitted.

In step ST201, whether or not to execute tracking processing of the X-ray diaphragm is selected. For example, selection is made by the user via the input interface 24.

In the case where the tracking processing of the X-ray diaphragm is to be executed (that is, in the case of YES) in step ST201, the processing advances to step ST202. In the case where the tracking processing of the X-ray diaphragm is not to be executed (that is, in the case of NO) in step ST201, the processing advances to step ST206. In step ST206, the user manually moves the X-ray diaphragm via the switches 244 and 245 for setting the position of the X-ray diaphragm in the input interface 24, for example.

In step ST202, the initial position setting function F01 sets an initial position of the X-ray diaphragm with respect to the X-ray tube 31 such that the predetermined part of the object is irradiated with the X-rays through the aperture.

In step ST203, the tracking start determination function F02 starts the tracking processing of the X-ray diaphragm. The tracking start determination function F02 determines the start of the tracking processing based on at least one of the operation state of the X-ray fluoroscopy to the object P and the fluoroscopic image obtained by imaging the object P. The tracking processing of the X-ray diaphragm may be manually started or may be automatically started. Tracking start processing of the X-ray diaphragm will be described with reference to Fig. 15.

In the case of the manual start, for example, when the user performs an operation of setting the switch 246 for the tracking of the X-ray diaphragm to the manual start, the tracking processing of the X-ray diaphragm is started (manual start 1 in Fig. 15).

In the case of the automatic start, the tracking start determination function F02 detects that at least one of the top plate 51 and the video system 36 has been moved during the period of the X-ray fluoroscopy, and starts the tracking processing according to the detection of the movement. For example, the tracking start determination function F02 may start the tracking processing when the X-ray diaphragm is not manually moved for the predetermined period after the X-ray fluoroscopy is started (automatic start 1 in Fig. 15).

The tracking start determination function F02 may start the tracking processing when the X-ray fluoroscopy is continued for the predetermined period in the state where the top plate 51 and the video system 36 are not moved (automatic start 2 in Fig. 15). The tracking start determination function F02 may start the tracking processing when the pulse rate is switched in the case where the X-ray fluoroscopy is operated in the pulse fluoroscopy mode (automatic start 3 in Fig. 15). Further, the tracking start determination function F02 may start the tracking processing when the dosage of the X-ray fluoroscopy is switched (automatic start 4 in Fig. 15) .

In addition, when a predetermined medical device is detected in the LIH image of the first X-ray fluoroscopy, the tracking start determination function F02 may start the tracking processing at the time of starting the second X-ray fluoroscopy following the first X-ray fluoroscopy (automatic start 5 in Fig. 15). Further, when a predetermined organ or tissue is detected in the LIH image of the first X-ray fluoroscopy, the tracking start determination function F02 may start the tracking processing at the time of starting the second X-ray fluoroscopy following the first X-ray fluoroscopy (automatic start 6 in Fig. 15).

In step ST204, when at least one of the top plate 51 and the video system 36 is moved, the tracking control function F03 moves the position of the X-ray diaphragm with respect to the X-ray tube from the initial position to track the predetermined part such that the predetermined part of the object P is continuously irradiated with the X-rays. The tracking control function F03 may perform the tracking processing by the arithmetic operation reflecting the TOD which is the distance between the predetermined part of the object P and the top plate.

In step ST205, the tracking control function F03 stops the tracking processing of the X-ray diaphragm. The tracking processing of the X-ray diaphragm is manually stopped. For example, when the user sets the switch 246 provided for the tracking of the X-ray diaphragm to the tracking stop, the tracking processing of the X-ray diaphragm is stopped.

The X-ray diagnostic apparatus 1 according to the third embodiment makes it possible to suppress the halation of the X-ray images and the exposure of the object while reducing the operation burdens caused by the adjustment of the X-ray diaphragm even in the case where the top plate and the video system are moved during the X-ray fluoroscopy or during X-ray radiography.

As an examination example to which the embodiments described above are applied, the examination example having the lung included in the X-ray image has been described, such as bronchoscopy and endoscopic retrograde cholangiopancreatography (that is, ERCP). Furthermore, the embodiments are also applicable to the examination example having a direct line on a chest side face included in the X-ray image, such as myelography, colonoscopy, and root block.

According to at least one embodiment described above, it is possible to suppress the halation of the X-ray images and the exposure of the object while reducing the operation burdens caused by the adjustment of the X-ray diaphragm and the compensation filter even in the case where the top plate and the video system are moved during the X-ray fluoroscopy or during the X-ray radiography.

In the above-described embodiments, the term "processor" means a circuit such as a special-purpose or general-purpose CPU (Central Processing Unit), a GPU (Graphics Processing Unit), an ASIC (Application Specific Integrated Circuit), a programmable logic device including an SPLD (Simple Programmable Logic Device) and a CPLD (Complex Programmable Logic Device), and an FPGA (Field Programmable Gate Array), for example. When the processor is a CPU, for example, the processor implements various functions by reading in and executing the programs (the medical image processing program) stored in the memory. In addition, when the processor is an ASIC, for example, instead of storing the programs in the memory, the functions corresponding to the programs are directly incorporated as a logic circuit in the circuit of the processor. In this case, the processor implements the various functions by hardware processing in which the processor reads out and executes the programs incorporated in the circuit. Additionally or alternatively, the processor can implement the various functions by combining the software processing and the hardware processing.

Although a description has been given of the case where a single processor of the processing circuitry achieves the respective functions in the above-described embodiments, the processing circuitry may be configured by combining a plurality of independent processors so that each processor implements each function. Further, when a plurality of processors is provided, a memory for storing the programs may be provided for each processor or a single memory may collectively store the programs corresponding to the functions of all the processors.

While certain embodiments have been described, these embodiments have been presented by way of example only, and are not intended to limit the scope of the inventions. Indeed, the novel methods and systems described herein may be embodied in a variety of other forms; furthermore, various omissions, substitutions and changes in the form of the methods and systems described herein may be made without departing from the scope of the inventions as defined by the appended claims.

## Claims

1. An X-ray diagnostic apparatus (1) comprising:
a top plate (51) on which an object is to be placed;
a video system (36) that includes an X-ray tube (31) and an X-ray detector (34);
a compensation filter (331) configured to attenuate X-rays irradiated from the X-ray tube (31) toward the object and to be movable from a position with respect to the X-ray tube (31); and
processing circuitry (21) configured to;
set an initial position of the compensation filter (331) with respect to the X-ray tube (31) to cover a predetermined part of the object, and
move the position of the compensation filter (331) with respect to the X-ray tube (31) from the initial position to track the predetermined part in such a manner that the compensation filter (331) keeps covering the predetermined part of the object when at least one of the top plate (51) and the video system (36) is moved.

2. The X-ray diagnostic apparatus (1) according to claim 1,
wherein the processing circuitry (21) is configured to automatically set the initial position of the compensation filter (331) based on a line segment set to an X-ray fluoroscopic image or an X-ray radiographic image of the object.

3. The X-ray diagnostic apparatus (1) according to claim 2,
wherein the processing circuitry (21) is configured to determine a region to be covered with the compensation filter (331) based on a luminance value of a region of the X-ray fluoroscopic image or the X-ray radiographic image divided by the line segment, and automatically set the initial position of the compensation filter (331) to cover the region.

4. The X-ray diagnostic apparatus (1) according to claim 1,
wherein the processing circuitry (21) is configured to determine an anatomical part where exposure should be reduced or a suppression part where halation should be suppressed, by using at least one of a result of image recognition to an X-ray fluoroscopic image or an X-ray radiographic image of the object and an imaging target part set beforehand, and set the initial position of the compensation filter (331) to cover the determined anatomical part or suppression part with the compensation filter (331).

5. The X-ray diagnostic apparatus (1) according to any one of claim 1 to claim 4,
wherein the processing circuitry (21) is configured to detect that at least one of the top plate (51) and the video system (36) has been moved during a period of X-ray fluoroscopy, and start tracking processing according to detection of the movement.

6. The X-ray diagnostic apparatus (1) according to any one of claim 1 to claim 4,
wherein the processing circuitry (21) is configured to start tracking processing when the compensation filter (331) is not manually moved for a predetermined period after X-ray fluoroscopy is started.

7. The X-ray diagnostic apparatus (1) according to any one of claim 1 to claim 4,
wherein the processing circuitry (21) is configured to start tracking processing when X-ray fluoroscopy is continued for a predetermined period in a state where the top plate (51) and the video system (36) are not moved.

8. The X-ray diagnostic apparatus (1) according to any one of claim 1 to claim 4,
wherein the processing circuitry (21) is configured to start tracking processing at the time of starting second X-ray fluoroscopy following first X-ray fluoroscopy when a predetermined medical device or a predetermined organ or tissue is detected in a Last Image Hold image of the first X-ray fluoroscopy.

9. The X-ray diagnostic apparatus (1) according to any one of claim 1 to claim 8,
wherein the processing circuitry (21) is configured to restart tracking processing with a position of the compensation filter (331) after adjustment as a new initial position in a case where the position of the compensation filter (331) is manually adjusted during the tracking processing.

10. The X-ray diagnostic apparatus (1) according to any one of claim 1 to claim 9, further comprising
a display,
wherein the processing circuitry (21) is configured to cause the display (23) to display an X-ray fluoroscopic image or an X-ray radiographic image of the object and a schematic diagram showing the compensation filter (331).

11. The X-ray diagnostic apparatus (1) according to claim 10,
wherein the processing circuitry (21) is configured to display the compensation filter (331) in different forms in the schematic diagram between a time when tracking processing is being performed and a time when the tracking processing is not being performed.

12. The X-ray diagnostic apparatus (1) according to claim 10 or claim 11,
wherein the processing circuitry (21) is configured to display the compensation filter (331) in the schematic diagram in a form that can be contrasted with a position of a visual field.

13. The X-ray diagnostic apparatus (1) according to any one of claim 10 to claim 12,
wherein the processing circuitry (21) is configured to display, in a case where the compensation filter (331) includes a plurality of compensation filters, the compensation filters in the schematic diagram which shows a relative positional relation among the plurality of compensation filters.

14. The X-ray diagnostic apparatus (1) according to any one of claim 10 to claim 13,
wherein the processing circuitry (21) is configured to display at least one of a moving direction of the compensation filter (331) and a rotation radius when the compensation filter (331) is rotationally moved in the schematic diagram.

15. A method of controlling an X-ray diagnostic apparatus (1),
the X-ray diagnostic apparatus (1) including:
a top plate (51) on which an object is to be placed;
a video system (36) including an X-ray tube (31) and an X-ray detector; and
a compensation filter (331) configured to attenuate X-rays irradiated from the X-ray tube (31) toward the object and to be movable from a position with respect to the X-ray tube (31),
the method comprising:
setting an initial position of the compensation filter (331) with respect to the X-ray tube to cover a predetermined part of the object, and
moving the position of the compensation filter (331) with respect to the X-ray tube from the initial position to track the predetermined part in such a manner that the compensation filter (331) keeps covering the predetermined part of the object when at least one of the top plate (51) and the video (36) system is moved.
